# EUROPEAN PATENT APPLICATION

(11) **EP 1 248 041 A2**
(43) Date of publication of application: **09.10.2002**
(21) Application number: 01309047.7
(22) Date of filing: 24.10.2001
(51) Int. Cl.: F23G 1/00, F23G 5/033, F23G 5/04, F23G 5/30, F23G 5/32, F23G 5/46, F23G 7/10

(54) **Thermal annihilation process**

(30) Priority: 04.04.2001 CL 2001771
(71) Applicant: Fernandez Mesa, Adolfo, Quilicura, Santiago (CL)
(72) Inventor: Fernandez Mesa, Adolfo, Quilicura, Santiago (CL)
(74) Representative: Carpintero Lopez, Francisco

(57) **Abstract**

The present invention discloses a process and equipment for the complete thermal annihilation of proteins contained in material of animal or vegetable origin, in solid or semisolid state, that comprises the steps of: (a) previously milling the material of animal or vegetable origin to assure that the drying step reaches to be completed in the whole material, without remaining areas or pieces with high humidity that can not reach high temperatures by being shielded from the heat; (b) drying the whole material to be able to increase the combustion temperature of the solids from 800°C to 1350°C; (c) thermally annihilating the material, by means o f a combustion in a refractory oven, either shredded or powdered and in suspension, to achieve the high required temperature to be reached and kept within the whole material.

## Description

### FIELD OF THE INVENTION

The present invention relates to a process for complete destruction of substances containing dangerous proteins for health, such as viruses or prions. The process is composed of shredding of vegetable or animal material containing viruses or prions, followed by a drying step, then a full thermal destruction of the vegetable or animal material, and optionally a further step of heat recovery which is a non necessary step for carrying out the process of thermal destruction obtained in the previous steps.

### BACKGROUND OF THE INVENTION

State of the art, regarding the methods used in destruction of substances containing dangerous proteins for health, such as viruses or prions, is extremely critical since specialists strongly believe in two concepts:
a) The waste dumping grounds destroy in a short term any possible focus of dangerous infection for health. We have found however, antecedents where the virus of AIDS remains active for one week inside the needles of used syringes treated with the normal sterilization processes actually used for; and
b) By means of incineration the prion of the spongiform encephalopathy, also known as the mad cow disease, is destroyed. Lately however it has been proved that prions remain active even after the waste containing them is exposed to temperatures of 600 °C; therefore its deposit into a waste dumping ground is a potential risk of a direct or indirect contamination of animals and plants for further transmission.

Unfortunately no technical norm has been found, after an exhaustive search, specifying the required conversion level of oxidation for the substances contained in the ashes of an incineration.

There is not standard regulations for characteristics the ashes of an incinerator should fulfill to be put into a waste dumping ground. In incinerator facilities operating in Canada, England, Switzerland and France it can be seen that the ashes contain an appreciable quantity of material of high humidity non burn during the oxidation process. The existing norms refer at large only to exhausted gases.

Regarding the result of total and permanent inactivation of organic solid materials, definitions used in the technical literature of incineration are not precise enough. For instance:
Incineration: it means to degrade waste thermally by way of a controlled flame, without indicating their final state, total oxidation by use of high temperature, what doesn't assure their final state of deactivation.
Calcination: it means to produce a change in a waste by means of heat, without fusing it, what doesn't indicate their final state of deactivation.

For a better understanding of this invention, the term "thermal annihilation" is proposed for the total and complete destruction of proteins through the application of high temperatures in an oxidant environment.

### STATE OF THE ART

State of the art referred mainly to incineration shows a great number of publications and/or elimination of waste.

ES patent number 2,003,963 to KUO, TSUNG-HSIEN, titled: " Method of waste incineration discloses a method of waste incineration in which wet residuals are heated in a dryer with incoming overheated vapor to eliminate most of the humidity of the residuals where it becomes exhausting vapor also in an overheated state. Then the dried residuals are fed to a boiler, through which circulate overheated vapor to increase the temperature at a higher level. This system increases the drying effectiveness and productivity, and also reduces the necessary volume of the dryer.

This publication doesn't have as objective the incineration of vegetable or animal materials, but rather it uses that materials as fuel to generate overheated vapor of high pressure to produce mechanical work. The material disclosed in this publication also has as objective to produce overheated vapor of low pressure to heat the input of water to the boiler.

US patent 4,905,614 to KLOCKNER HUMBOLDT DEUTZ AG, titled: "Procedure for the thermal elimination of residuals" points out toward thermal elimination of residuals like muds, special or similar garbage using techniques of incineration of garbages with grill home; it is polluting and not very profitable. To get a profitable and non pollutant elimination of residuals, it proposes to crumble the residuals and to dry them by means of preheating in a hurricane oven and fire residuals above 1500 °C in the hurricane oven or combustion hurricane to obtain some heated exhaust gases and fused flowing scum containing noxious substances; then, noxious and valuable materials are vaporized from the residuals. From the exhausting gases partial conduction separates valuable and noxious powder materials.

The subject material of this publication is to obtain fused ashes, reaching temperatures of 1500 °C or even more, without indicating contribution of the fuel. Also this looks for aglomeration of ashes but not for the annihilation of proteins.

EP 0423400 to DEUTSCHE VOEST ALPINE INDUSTRIEANLAGENBAU GMBH, titled: "Facility of garbage elimination", discloses a facility for the elimination of aqueous residuals, for instance mire from water decantation, humid residuals from gardening or similar materials that generates the necessary vaporizing energy for drying, or part of it through combustion of the solid particles of gross materials. Installation is made up of a mechanical pre-dryer (3), a fluid bed dryer (4), a separator of solid materials (12), a burner of solid materials (6), a separator of ashes (5) and a heat exchanger (7). Drying in fluid bed and combustion of the solid particles with the corresponding separators, fans (8, 9), and distributors are gathered together into a single compact unit that is mounted on a vehicle. Mechanical pre-dried is carried out separately, in a trailer (2).

EP 0692679 to DEUTZ AKTIENGESELLSCHAFT, titled: " Method and plant for the drying by centrifugation of muds and their later incineration in a process of pulsant combustion", describes a method and a plant for the combustion according to the environment of deposited mud.

The dried mud is conducted with reduced cost and with water elimination in a procedure by means of a centrifugal drier (1) in a mechanical way, being controlled without another intermediate crumbling and guided to a pulsed reactor (13). At temperatures above 850 and with times of residence in the secondary area, harmful materials and organic scent undergoes almost complete combustion, while exhausting gases are used starting from the pulsation combustion by means of indirect exchange as drying energy.

WO 90/12249 to SAARBERGWERKE AKTIENGESELLSCHAFT, titled "Procedure for using the mud of decantation", discloses a method for reprocessing sewer mires, in which the mire is dried before burning; it proposes to use vapor of the circuit of water vapor from a power energy station fed with fossil fuels as mean of heating for the drying and to take vapors coming out during the normal drying down stream the home of the oven of the energy power station to reduce nitrogen oxides. Preferably the dried sewer mires burn together with coal in the oven of the power energy station.

As a whole, all the documents of the previous art, attempt waste incineration, but none of them mentions the " thermal annihilation " for the total and complete destruction of proteins by means of the application of high temperatures in an oxidant environment and in agreement with specifications that follow. With the present invention the total annihilation of the prions, bearing the information of the "mad cow disease", is obtained. This remarkable fact of the invention can not be found in any of the documents of the previous art mentioned above. The fact of burning dead animals as granulated or powdered dried material until a temperature of 1100 °C, and preferable 1350 °C, to totally eliminate the organization information that produces mad cow disease is not deduced in an evident way, not even combining some of the documents of the previous art already discussed.

### DESCRIPTION OF THE INVENTION

Considering what was explained before, the use of the process and the equipment of the present invention, is oriented to produce total oxidation of ashes, with the aim of getting thermal annihilation of proteins such as viruses and prions, in such a way that the residuals can be placed under safe conditions in any waste dumping ground.

### Technical concepts to be used in the new system

The fundamental factors involved in the thermal annihilation of the proteins under discussion are:
- Temperature
- Time
- Oxygen Concentration

### These factors are involved in the following aspects:

### Drying.

Without this previous operation the material doesn't arrive at a temperature of approximately 100 °C although it is subjected to a higher environmental temperature. As can be seen in the calculation of the combustion of an animal without previous drying, from Table 1, the maximum temperature of the gases of combustion, in a closed special system and with a strict control of the air in excess, is about 800 °C. It is very probable that temperature of the wet solid present during the combustion doesn't overcome 110 °C, both if the solid is on a grill through which the combustion air is introduced, or if it is occasionally shaken in a fixed bed.

**Table 1**

| **Combustion of an animal without previous drying** | | |
|---|---|---|
| **Wet Original Material** | | |
| **COMPOSITION** | | |
| Element | | % wet base |
| C | | 14.70 % |
| H | | 2.45 % |
| O | | 11.75 % |
| N | | 0.00 % |
| S | | 0.00 % |
| H₂O | | 62.10% |
| Ashes | | 9.00 % |
| **Total** | | **100.00 %** |
| | | |

| **GLOBAL COMPOSITION** | | |
|---|---|---|
| **Component** | | **%** |
| | | |
| WATER | | 62.10 % |
| FUEL | | 28.90 % |
| ASHES | | 9.00 % |
| **TOTAL** | | **100.00 %** |
| | | |

| **Caloric Power** | | |
|---|---|---|
| PCS (Kcal/Kg) | 1.416 | |

| **Combustion gas Temp.** | **°C** | |
|---|---|---|
| | **1073** | |

| **COMBUSTION PRODUCTS** | | |
|---|---|---|
| % vol d. b. | | |
| CO₂ | 13.8 % | - |
| NO | 0.0 % | - |
| SO₂ | 0.0 % | - |
| N₂ | 80.7 % | - |
| O₂ | 5.5 % | - |

### Conduction of the heat toward the interior of the pieces or particles.

For the material submitted to the thermal annihilation, to achieve the required temperature, it should receive heat contribution from its environment. Doing a simplified analysis, it is possible to conclude that the heat contribution of a spherical particle with a particular diameter (two times a radius R) having a mass proportional to (4/3 π R³), it is carried out through their surface (4 π R²), and it should travel a distance similar to the radius (R) to get the center. That means, for a certain heat transmission speed, by conduction, the time that it takes in warming the interior is proportional to the radius of the spherical piece, (R). Therefore, the effect of the time required to reach a certain level of temperature is strongly influenced for the size of the pieces, being the heating process faster, if a certain quantity of the material is finely milled previously to the heating. A very similar phenomenon occurs during the drying operation with regard to the time required and the diffusion speed of the water in the solid.

### Threshold temperature

The oxidation processes require certain activation energy and therefore, they won't begin unless an appropriate level of temperature is achieved, in the material itself, which is not necessarily the environmental temperature.

### Time to complete the process.

The thermal annihilation involves several kinetic phenomena of transport:
- Diffusion and evaporation of the water during the drying.
- Diffusion of the heat required to reach the activation temperature of the oxidation reactions.
- Diffusion of the oxygen toward the interior of the pieces.
- Diffusion of the gaseous combustion products, CO₂ and H₂O, from the interior of the pieces toward their external environment.

All these phenomena of transport are not instantaneous, so they require a minimum time for them to happen and be completed. As previously indicated, that time is strongly influenced by the size of the pieces and their homogeneity. If there are pieces of very diverse sizes, in a certain time or condition, the smaller ones will be completely oxidized and the bigger ones will only be partially oxidized.

Therefore, for a correct design of the required equipment and a sure result of the process, it is critical to previously control the size of the pieces or particles. When the equipment is a cyclonic oven, then it will be necessary to mill the material, after the drying step and before the combustion step. In this case the size of particles is below 1 mm. When the equipment is an oven of long time of residence, *i.e.,* a rotational oven, then bigger pieces of material will be able to full combustion.

### Operations that the process of the invention should fulfill and their comparison with other current techniques.

### Size reduction

To get a higher reactivity and a more homogeneous behavior of the material, the size of the initial pieces which can be a whole complete animal, should be drastically diminished. Due to the very high content of humidity that is normally found in insects, animals or plants, the use a shredder or a shearer allowing a problem free operation is recommended. Later on, in between the drying step and that of oxidation, it is convenient to use a capable crusher for dry materials.

Until now, in the state of the current technique, this operation of size decreasing has been applied to get an easier handling of the wastes for incineration, but not as a requirement for an appropriate drying and combustion.

### The pre-drying previous to combustion.

To overcome the limited temperature raising of the solid to be annihilated due to the high content of water and to standardize the time of processing for all the pieces, the material should be dried.
There are basically three types of dryers:
   - The indirect or conductive ones, in which the solid is heated through metallic walls, by means of vapor at a high pressure. Very voluminous and very robust equipments are required due to the long time needed for heat transmission by conduction and also they should be very robust due to the high resistance needed as a consequence of the high pressure created by the means of heating. These dryers evacuate vapors at atmospheric pressure almost without air, in such a way that the heat can be recovered and almost no polluted gases are emitted.
   - The direct or convective ones, where heat contribution is made through hot air or hot gases. They operate at atmospheric or similar pressure. They are faster and more economic equipments where the heat used can't be recovered and also they generate a great volume of polluted gases for later treatment.
   - The Overheated Vapor dryer, contributes heat by a convective way by means of overheated vapor, where emission of polluted gases is not produced, all of the heat used in drying is recovered and the equipment is quite light since it operates at atmospheric pressure.

### Combustion at high temperature.

For a better control of its total oxidation it is convenient that the granulated dried material be finely milled. It is also convenient that it be burned in suspension, so that all the particles be completely exposed to the oxygen and to high temperature. The residence time in a high temperature combustion chamber lined with refractive material is regulated, with values higher than 2 seconds. It is not acceptable for vapor generation to use wet wall incineration chambers as it is done in the current technique, since such walls locally limit temperature to boiling water temperature which is quite lower than the one required for starting and keeping the total oxidation. On the other hand, the fixed combustion beds, although they are shaken, they suffer of an uncontrolled and insufficient exposition of the material to the oxygen and to the high temperature.

In this invention the proteic material that will be exposed to destruction can not be used as a fuel in a vapor generator or a fluid heater, even if it is already dried. The low temperature of the walls prevents to reach the level of temperature required by the thermal annihilation. Heat recovery from gaseous products of the proposed process can be done with any of the systems commonly known, but only after having completed the thermal annihilation process and having left the refractory oven.

Therefore, the Thermal Annihilation Process requires the combustion of the shredded or powdered material with the much reduced size possible, for controlling the required oxygen excess needed for an oxidizing atmosphere and for secure reaching of a temperature and an appropriate time for the whole material. For that the powdered material should have preferably less than 1 mm.

It is appropriate to emphasize, according to what has been exposed, that incineration in graves of piled animals is a mistake, since:
- The high humidity of the animals that remain at the bottom prevents that its temperature increases due to the low amount of heat coming down from those that are burning on the surface of the pile (the heat ascends by convection, but it never descends).
- The water vapor has a specific heat whose value is almost twice of the other gases involved in the combustion, so evaporation of the water from the material located in the inferior part of the pyre, is a barrier for the temperature that can achieve the material already dried burning at the surface. By this reason, the dry material under combustion does not reach the required conditions for thermal annihilation.
- After the pyre is finished, even when in the process some type of fuel has been added, in the inferior part remain areas with unburn wet material with a significant content of proteins not thermally annihilated, and that means dangerous material remains. According to what has been pointed out in the previous paragraphs, the present invention considers four necessary steps for carrying out the thermal annihilation process.
   a) Previous size reduction of the material to assure the drying step is completed over the whole material, without remaining areas or pieces with high humidity which can not reach high temperatures since they are shielded from the heat.
   b) Drying of the whole material, to increase the combustion temperature of the solids for reaching 1100 °C, preferably 1350 °C. As it was pointed out previously, for the drying it is possible to use different dryers: a) indirect or conductive dryer; b) direct or convective dryer; or/and preferably c) overheated vapor dryer.
   c) Combustion of the material into a reactor or refractory oven, milled or preferably powdered and in suspension, to achieve that the required high temperature be reached and kept on the whole material, that a homogeneous concentration of oxygen be present, and that it can be assured that the time of treatment allows getting the thermal annihilation. In this step it is possible to reach temperatures of 1350 °C or more, depending on the origin of the animal material (bovine, pig, sheep, etc.), without adding fuel. Nevertheless, in this step a second burner can be used, to avoid temperature variations. It is important to remember that the material should remain in the reactor of the step (c) a higher time than the required one to get completion of thermal annihilation reactions; being this required time, that one the chemical reactions of oxidation involved in the thermal annihilation take for completion.
   d) The recovery of the heat of combustion of the material should be carried out after the total oxidation of it, but never during its combustion, to avoid decreasing of temperature before the treatment has been completed.

The drying with overheated vapor, is a drying of convective type that uses overheated vapor, which comprises the steps of: (a) introducing said animal material into a camera of controlled atmosphere free of air or another gas; (b) circulating overheated vapor through said camera, and (c) keeping humidity of the product. under control.

### Description of the drying with overheated vapor

Although it is true, the process of thermal annihilation can be carried out by any drying method, a preferable embodiment of the present invention is drying with overheated vapor.

The drying method for materials containing proteins, which is preferred in the present invention, it doesn't use boiler vapor neither air or combustion gases as most of the actually well-known methods do. The method uses direct vapor, heated beyond their saturation temperature, that means overheated vapor, as heat provided to the solid that will be dried.

Although drying with overheated vapor is known for several applications, in none of them a careful and fine control of the humidity and process is required since the materials here involved are very thermo sensitive substances of animal origin. For that reason there is no standard ,method about control of the process like the one required for an appropriate drying of proteins. This drying method here established provides the way to achieve the control of required drying.

### Theoretical bases of the drying with overheated vapor

State of the art shows that the more used drying method is the concept of convective drying associated to the use of hot air. This means the room temperature of the air with an absolute humidity in the order of 0,01 Kg of Water/Kg of dry air is raised. With this operation partial pressure of water vapor in the air sharply decrease in relation to which it would have if it is saturated, that means their relative humidity decreases generating hot air that needs water. Technically the humid solid would be dried since their temperature on their surface generates a vapor pressure higher than the differential of water vapor pressure of the hot dry air regarding their saturation. This common daily concept of drying makes not easy to explain why it allows to use overheated vapor since this already contains pure water, so it would have not potential for drying since its vapor pressure is similar to the total pressure of the system.

The method puts in contact proteic materials with overheated vapor, within some equipment that avoid the access of air such as a chamber of controlled atmosphere free of air or another gas, during the necessary time for the drying process to be completed. Drying will depend on the temperature of the solid, and the present atmosphere of water vapor will not interfere while the pressure of the system is maintained.

For understanding the basics of the concept of this part of the invention it has been necessary to elaborate an approach that even when using well-known concepts, specifically it work with them in different ways.

Gases or vapors move from the points of higher pressure toward the points of lower pressure with a global movement as it was free wind. If into a gaseous atmosphere made out of several components a concentration difference does exists at different points, then a diffusion will take place within molecules from the points of higher concentration (or higher partial pressure), toward the points of lower concentration (or lower partial pressure). It is important to understand that a gas or a vapor of a single component has a partial pressure equal to the total pressure, that means the pressure of the atmosphere where it is involved.

If water evaporates into a device of closed but variable volume, without another gas, at room pressure, a temperature of 100 °C will be kept while liquid water is still present. But if heat continues to flow in after total evaporation, vapor will be overheated. Then, if this overheated vapor is put in contact with a substance to lower temperature, heat will be transmitted to it until their temperatures are equalized, without condensation, as long as the temperature of 100 °C is not reached. If that substance is water a part of it will evaporate until it get an average temperature of 100 °C in the whole system.

On the other hand, if into a device of constant volume is put under vacuum an amount of a wet solid, and temperature is increased, the pressure of the system will also increase. The wet solid also has a characteristic vapor pressure for each temperature. If the water has only moistured the surface of the solid, the vapor pressure will correspond to the pure water at that temperature. If the solid has been moistured in its interior, the water can be caught by capillarity, adsorption or chemically, so the vapor pressure will be lower than the pure water.

In the preliminary investigation of this drying method it was possible to establish that meat or fish flours have a characteristic vapor pressure, lower than the pure water, what is critical for handling the equilibrium reached, that means the final humidity of the product. It is important to realize that at an atmosphere of pure vapor, the dependence of the output product humidity referred to the temperature of the effluent overheated vapor is much more direct that in the case of using gases or hot air where several variables have influence (such as input temperature of air, input temperature of the wet solid, absolute humidity of the hot air, caloric losses in the equipment, leakage of air through the seals, etc.), getting a much more effective and direct control of the drying.

The quantity and/or temperature of the overheated vapor is regulated in such a way that the temperature of the product be the one in which, humidity reaches the vapor pressure of the system, preferably the atmospheric pressure. In an equipment of appropriate design the temperatures of the solid and the vapor, even overheated, should be equal or very similar to each other, so both variables allow to control the process, being the quickest and the one that allows better control, the effluent vapor overheated.

During calculation of the overheat temperature required for the vapor, it is important to consider that the specific heat of the water vapor is approximately 0,44 Kcal/Kg/°C versus the approximate specific heat of 0,27 Kcal/Kg/°C of the air or the combustion gases that is conventionally used as heating gas, so the required temperature will be lower with this drying method.

On the other hand, if the temperature of 100 °C in the material to be dried is too high for certain requirements of quality of the product, in spite of the anaerobic drying, the temperature of this material can decreases by using another volatile substance that decrease the partial pressure of water vapor in the mixture of overheated vapors which are dried with. It is important to consider that if instead of a volatile substance air or another gas non condensable is introduced, the system will work conceptually in a similar way, but it will loose many of their advantages. The same would happen if as a consequence of a design with not very effective seals leakage of air are produced

### Practical use

The overheated vapor can be obtained from an adiabatic expansion of saturated vapor of high pressure, of a boiler of overheated vapor or overheating vapor of another source by means of a heat exchanger of indirect type, according to the desired temperature. It is especially interesting the possibility that the method offers of overheating vapor to a pressure near to room pressure through an indirect exchanger getting high temperatures with low pressure equipment of lower cost and lower risk than the ones required for indirect drying. In this indirect exchanger, according to the wanted temperature, it is possible to use vapor of high pressure, thermal fluid or combustion gases. At the starting of the process air can be used, which will disappear through the exhaustion of what is in excess.

The equipment where the process is carried out should allow for separation of dried product from the exhausting overheated vapor, with no air contact, otherwise an immediate condensation will be produced. Similarly, evacuation of the solid should not allows air to get into the dryer.

The evacuated vapor, slightly overheated, can be used in any other equipment requiring to reach 100 °C, since it has a good coefficient of heat transfer due to the absence of non-condensable material.

### Application example of the invention

The following example in table 2, shows the incineration of a mammal in humid base, in which is demonstrated that the temperature doesn't reach the necessary temperature to annihilate prions. The animal on a dry base instead (with a previous pre-drying step) reaches a temperature of 1350 °C allowing thermal annihilation of prions.

**Table 2**

| Example of incineration of a humid mammal versus thermal annihilation of a dried mammal | | | | |
|---|---|---|---|---|
| | Example with | **10.000** | Kg/Hr | |
| | Wet original Material | | Dry Material | |
| **COMPOSITION** | | | | |
| **Element** | **% wet b.** | **wet Kg** | **% dry b.** | **dry Kg** |
| C | 14,70 % | 1.47011 | 38,79 % | 1,470.1 |
| H | 2,45 % | 245,0 | 6,48 % | 245,0 |
| O | 11,75 % | 1,174,9 | 31,00 % | 1,174,9 |
| N | 0,00 % | - | 0,00 % | - |
| S | 0,00 % | - | 0,00% | - |
| H₂O | 62,10% | 6,210,0 | 0,00 % | - |
| Ashes | 9,00 % | 900,0 | 23,75 % | 900,0 |
| **Total** | | **10,000,0** | | **3,790** |

| **GLOBAL COMPOSITION** | | | | |
|---|---|---|---|---|
| | | **Wet Base** | | **Dry base** |
| | **%** | **Kg/Hr** | **%** | **Kg/Hr** |
| WATER | 62,10 % | 6,210,0 | 0,00 % | - |
| FUEL | 28,90 % | 2,890,0 | 78,25 % | 2,890,0 |
| ASHES | 9,00 % | 900,0 | 23,75 % | 900,0 |
| **TOTAL** | | **10,000,0** | | **3,790,0** |

| **Caloric Power** | | | | |
|---|---|---|---|---|
| | PCS(Kcal/Kg) 1,416 | | PCS(Kcal/Kg) 3,737 | |
| **Temp. Gas combustion C** | | **800** | | **1,375** |

| **COMBUSTION PRODUCTS** | | | | |
|---|---|---|---|---|
| | **% vol d. b.** | | **% vol d. b.** | |
| CO₂ | 13,8 % | 5,386.5 | 13,8 % | 5,386.5 |
| NO | 0,0% | - | 0,0% | - |
| SO₂ | 0,0% | - | 0,0% | - |
| N₂ | 80,7 % | 20,225.0 | 80,7 % | 20,225.0 |
| O₂ | 5,5% | 1,409.9 | 5,5% | 1,409.9 |
| Dry gases | | **27,021.5** | | **27,021.6** |
| Water vapor | | 8,876,5 | | 2,466.5 |
| Total humid gases | | 35,698.0 | | 29,488.0 |
| Dry air combustion | | 26,334.7 | | 26,334.7 |

### Advantages over the state of the art

The main advantage of this new process for destruction of proteins, is the security of completing the steps of the process, that means, the security that viruses and prions have been deactivated by the thermal annihilation.

The advantages of carrying out the Thermal Annihilation Process with a drying step with overheated vapor are the following:

The re-use of heat allows to diminish strongly the energy cost of fuels at the industrial plant, since it can be recovered with a very small additional expense in energy.

The heat used in the drying is recoverable in equipments of small size and high efficiency, due to condensation of vapor virtually saturated without the presence of non condensable gases

The use of this heat allows to diminish the investment in additional equipment for vapor generation such as boilers of higher cost than the recovery heat equipment of the previous paragraph.

Environmental pollution is totally eliminated; unpleasant smelling that normally goes with standard incineration is suppressed. The possible contamination of the effluent liquid is much simpler and economic of handling that of gaseous effluent, besides the amount of water to handle is substantially smaller than with effluents containing air.

The high speed of heat contribution to the drying solid, when being made by convection, allows high speed processing, facilitating quality control of the product, due to lower times of residence required, versus dryers of conductive type.

The high speed of heat contribution and the biggest exposed area to drying produce a high evaporation speed. The drying heat is provided by the present overheated vapor, so when evaporation goes on, it cools down equally fast, therefore times of exposition to relatively high temperatures are very short, compared to dryers of conductive type. This produces additional advantages from the view point of being able to preserve the quality of the product and being able to control the final humidity easily by means of regulation of the temperature of overheated vapor.

The drying method of this invention requires a smaller investment than the one for equipment of conductive drying, since the equipments can be smaller than conventional due to the high process speed and the low times of residence required. For this reason they have a smaller cost and their space requirements in the industrial plant are smaller to those of the other systems.

As the heat contribution is made with overheated water vapor, it should be considered that the specific heat of the water vapor it is of approximately 0,44 Kcal/Kg/°C against the approximate specific heat of 0,27 Kcal/Kg/°C of the air or the combustion gases conventionally used as heating gas. The analysis of the ratio of these values indicates that the quantity of heat can be contributed more easily with the well-known convective dryers and additionally the following points are obtained:
- decreasing to 61 % approximately, the necessary gradient of temperature of input gases, what allows to use quite smaller heat exchangers for vapor overheating.
- decreasing to 61 % approximately the flow mass of required gases, what allows to use even smaller equipments in the overheater and in the dryer with the consequent lower physical required space and lower cost.

Decreasing requirements of external to the process vapor, at the industrial plant, is possible to recycle only the necessary fraction of the exhausting vapor toward the exchanger overheater what allows to evacuate only that evaporated as available vapor for heating.

The proposed drying method allows for overheating vapor at a near to the atmospheric pressure by means of an indirect exchanger, with allows for getting high temperatures with a low pressure equipment, of a lower cost and risk than the equipment required for indirect drying.

The control method which is practically non existing in the conductive or indirect dryers and which is complex and imprecise in the convective or direct dryers, is extremely simple and accurate. It is enough just to control the exit temperature of the dry product or the temperature of the overheated vapor evacuated of the process.

Although it is certain that the Thermal Annihilation Process can be carried out by what is known in the art equipment, the grouping of these equipments to carry out the process is novel.

Indeed, the equipment to carry out a process of thermal annihilation of protein contained in materials of animal or vegetable origin comprises: (a) means to cut the animal or vegetable material and transform it into smaller pieces of homogeneous size, as for example: a Shredder cutter of low speed; a mill of rocking or fixed hammer of high-speed cutting; a slicer of high-speed, or a continuous screw mill of low speed cutting; (b) means to dry the cuted material, for instance: a convective direct dryer of overheated vapor; an indirect or conductive dryer with external heating or a direct or convective dryer with air or hot gases; (c) means to burn the crushed or powdered dried material, by combustion until reaching a temperature on 1100 °C, and preferably 1350 °C where these means to burn the dry material is an oven of refractory walls, as a suspension cyclonic oven; a fluid bed oven; a rotatory oven or a shaken fixed bed; and (d) means to recover the heat of combustion of the material.

The exhausting gases from the oven at high temperature, allow to use apparatuses of heat transfer of relatively small size, that means, more compact heat exchangers.

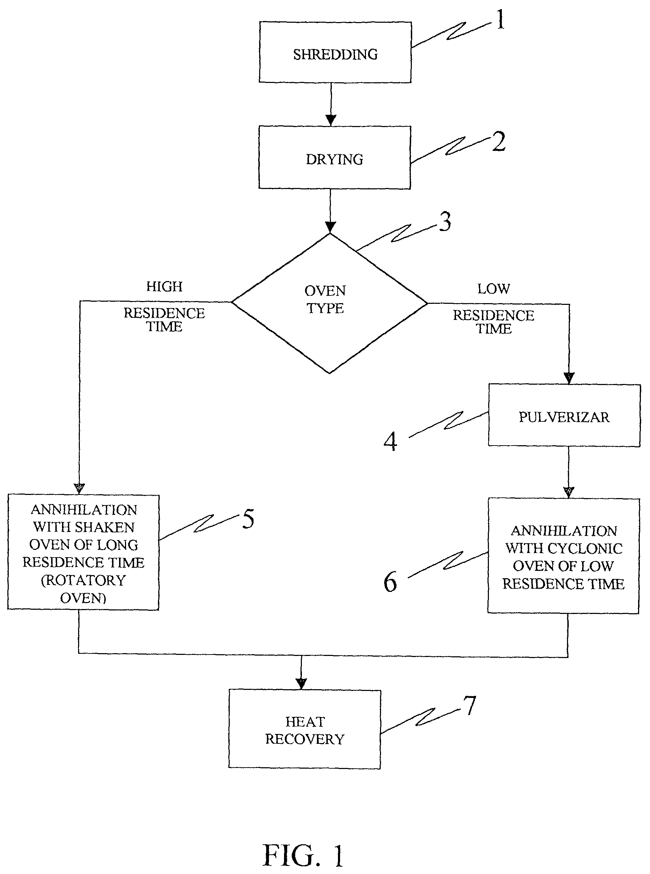

In the flow diagram of figure No. 1, is observed that the first step (1) it is the step of shredding the dead animal. Next, the second step (2) it is the drying of the shredded material. It is important to know what equipment will be used for the annihilation step (5, 6). In this case, discrimination is done in (3) for the oven type that will be used. If the residence time of the material in the oven is large, then it is not necessary a shredding step and the process continues directly to (5) up to the annihilation step which is carried out in a rotatory oven. If the residence time of the material in the oven is short, then a milling step (4) will be necessary to get the material with a particle size of less than 1 mm. Once milling is done, annihilation (5) will be done, which is carried out into a cyclonic oven. Finally in (7) the heat of combustion is recovered once the oxidation has been completed.

## Claims

1. A Thermal Annihilation Process for proteins contained in material of animal or vegetable origin, in solid or semisolid state comprising the following steps:
(a) shredding of materials of animal or vegetable origin for homogenizing the size of particles that will be subjected to drying;
(b) drying the whole material;
wherein the following steps are comprised:
(c) thermal annihilating the shredded material in step (a), by means of combustion into a refractory oven, until reaching a temperature on 1100 °C; and
(d) recovering the heat of combustion of the material, that should be made after the total oxidation of the material.

2. A Thermal Annihilation Process for proteins contained in material of animal or vegetable origin, according to claim 1, wherein shredded material in step (a) and dried in step (b) goes directly to annihilation step (c) when the material pieces are larger than 1 mm.

3. A Thermal Annihilation Process for proteins contained in material of animal or vegetable origin, according to the claim 1, wherein the material obtained in step (b) is previously powdered to a size smaller than 1 mm before going to the annihilation step.

4. A Thermal Annihilation Process for proteins contained in material of animal or vegetable origin, according to the claim 3, wherein in step (c) the powdered material is annihilated throuh a combustion in suspension until reaching the temperature 1100 °C.

5. A Thermal Annihilation Process for proteins contained in material of animal or vegetable origin, according to the claim 1, wherein shredding step (a) is done to assure that drying step (b), will be completed in the whole material, without remaining areas or pieces with high humidity that can not reach high temperatures by being shielded of heat.

6. A Thermal Annihilation Process for proteins contained in material of animal or vegetable origin, according to the claim 1, wherein during the drying step (b) the material is dried, for the combustion temperature of step (c) can be increased until reaching a temperature of 1350 °C, or more, according to the origin of the animal material.

7. A Thermal Annihilation Process for proteins contained in material of animal or vegetable origin, according to the claim 1, wherein in the step (c) the temperature of 1350 °C or more, can be obtained without any fuel addition.

8. A Thermal Annihilation Process for proteins contained in material of animal or vegetable origin, according to claims 1, 6 or 7, wherein in step (c) to get the required high temperature be reached and kept in the whole material, it is required that the oxygen concentration be homogeneous.

9. A Thermal Annihilation Process for proteins contained in material of animal or vegetable origin, according to claims 1, 6, 7 or 8, wherein in step (c) it can have an additional burner to avoid temperature variations.

10. A Thermal Annihilation Process for proteins contained in material of animal or vegetable origin, according to claims 1, 6, 7, 8, or 9, wherein the material should remain in the reactor of step (c) a larger time that the one required for the reactions of thermal annihilation be completed, being the required time, the time the chemical reactions of oxidation involved in the thermal annihilation take for being completed.

11. A Thermal Annihilation Process for proteins contained in material of animal or vegetable origin, according to claim 1, wherein step (d) should never be carried out during combustion of the material, for avoiding decreasing of the combustion temperature before the annihilation of the protein contained in the material has been completed.

12. A Thermal Annihilation Process for proteins contained in material of animal or vegetable origin, according to any of the claims 1 to 11, wherein drying step (b) comprises the following steps:
(b1) introducing said material in a drying container;
(b2) circulating evaporated water inside said container, heated beyond the existing boiling temperature of water, to make the heat contribution and the water evacuation from said material, in such a way of getting a dried product, being said evaporated water an overheated vapor;
(b3) controlling the environment of the container or drying camera, in such a way that said environment be free of air or another gas; and
(b4) transferring said dried material to an annihilation chamber.

13. A Thermal Annihilation Process for proteins contained in material of animal or vegetable origin, according to claim 12, wherein after the step (b2), it comprises a step (b2.1) consisting in adjusting the exit humidity of the material, by varying the amount of overheated vapor input, maintaining the temperature of input constant.

14. A Thermal Annihilation Process for proteins contained in material of animal or vegetable origin, according to claim 12, wherein after step (b2), it comprises a step (b2.1) consisting in adjusting the exit humidity of the material consisting in keeping constant the input amount of overheated vapor, by varying the input temperature.

15. A Thermal Annihilation Process for proteins contained in material of animal or vegetable origin, according to claims 12 or 14, wherein after the step (b2.1), it keeps under control the humidity of the material to the exit of the drying step, being only necessary to keep constant the temperature of the overheated vapor evacuated in said step.

16. A Thermal Annihilation Process for proteins contained in material of animal or vegetable origin, according to claims 12 or 14, wherein after the step (b2.1), it keeps under control the humidity of the material to the exit of the drying step, being only necessary to keep constant the temperature of said evacuated material in said step.

17. A Thermal Annihilation Process for proteins contained in material of animal or vegetable origin, according to claims 15 or 16, wherein in steps (b2) and (b2.1) overheated vapor is used without air or another gas to heat the material to be dried.

18. A Thermal Annihilation Process for proteins contained in material of animal or vegetable origin, according to claims 15 or 16, wherein in the drying step gaseous effluents go out, comprised only by water vapor, without requiring another gas or different vapor to produce the haulage and later evacuation of the evaporated material.

19. A Thermal Annihilation Process for proteins contained in material of animal or vegetable origin, according to claims 15 or 16, wherein in the drying step the evacuated gases are totally condensable, what allows to recover the heat of vaporization of the water drained off through its condensation in any other equipment using vapor.

20. A Thermal Annihilation Process for proteins contained in material of animal or vegetable origin, according to claims 15 or 16, wherein in the drying step the same flowing vapors can be recycled through a heat exchanger to generate the required overheated vapor.

21. A Thermal Annihilation Process for proteins contained in material of animal or vegetable origin, according to claims 15 or 16, wherein in the drying step to decrease the temperature of the solid material during the drying it can be added or recycled together with vapor another volatile liquid in the operation temperature range.

22. A Thermal Annihilation Process for proteins contained in material of animal or vegetable origin, according to claims I to 11, wherein the drying step (b) it is carried out through an indirect or conductive method.

23. A Thermal Annihilation Process for proteins contained in material of animal or vegetable origin, according to claims 1 to 11, wherein the drying step (b) it is carried out through a direct or convective method.

24. A Thermal Annihilation Process for proteins contained in material of animal or vegetable origin, according to claims 1 to 23, wherein the recovery of the heat of combustion is produced after the oxidation is completed.

25. An equipment to carry out a Thermal Annihilation Process for proteins contained in material of animal or vegetable origin, in solid or semisolid state, as the one described in claims 1 to 24, wherein it comprises:
means to cut the animal r or vegetable material and to transform it into smaller pieces of homogeneous size;
means to dry the cut material;
means to burn the dried shredded or powdered material, through a combustion until reaching a temperature over 1100 °C; and
means to recover the heat of combustion of the material.

26. An equipment according to the claim 25, wherein the means to burn allow to reach a temperature of 1350 °C or more, according to the origin of the animal material.

27. An equipment according to the claim 25, wherein the means to shred is in a Shredder of low speed.

28. An equipment according to the claim 25, wherein the means to shred is a mill of rocking or fixed hammer of high-speed cutting.

29. An equipment according to the claim 25, wherein the means to shred is a slicer of high speed.

30. An equipment according to the claim 25, wherein the means to shred is a continuous screw mill of low speed cutting.

31. An equipment according to the claim 25, wherein the means to dry is a direct convective dryer of overheated vapor.

32. An equipment according to the claim 25, wherein the means to dry is an indirect or conductive dryer with external heating.

33. An equipment according to the claim 25, wherein the means to dry is a direct or convective dryer through air or hot gases.

34. An equipment according to the claim 25, wherein the means to burn the dried shredded or powdered material is an oven of refractory walls.

35. An equipment according to the claim 34, wherein the oven is a suspension cyclonic one.

36. An equipment according to the claim 34, wherein the oven is a fluid bed.

37. An equipment according to the claim 34, wherein the oven is a shaken fixed bed.

38. An equipment according to the claim 25, wherein the gases that come out at high temperature allow to use heat exchangers of relatively small size.
